# EUROPEAN PATENT SPECIFICATION

(11) **EP 0 732 085 B1**
(45) Date of publication and mention of the grant of the patent: **08.10.2003**
(21) Application number: 96200680.5
(22) Date of filing: 13.03.1996
(51) Int. Cl.: A61F 2/06, A61M 29/02

(54) **Balloon catheter with light-conductive basic body**
Ballonkatheter mit Lichtleitgrundkörper
Cathéter à ballon avec corps de base conductif de lumière

(30) Priority: 15.03.1995 NL 9500516
(43) Date of publication of application: 18.09.1996
(73) Proprietor: CORDIS EUROPA N.V., NL-9301 LJ Roden (NL)
(72) Inventor: Leone, James Ernest, 33156 Miami, Florida (US); Ligtenberg, Hendrikus Cornelis Geert, 9351 PX Leek (NL); Haan, Marcel Gerard, 9312 PE Nietap (NL); Thalens, Jan, 9406 AW Assen (NL)
(74) Representative: 't Jong, Bastiaan Jacob

(56) References cited:
- EP-A- 0 446 178
- EP-A- 0 646 360
- WO-A-83/03188
- WO-A-94/24962
- US-A- 5 019 040
- US-A- 5 100 429
- US-A- 5 125 925

## Description

The invention relates to a balloon catheter comprising in the usual manner a tubular basic body with a proximal and a distal end. A balloon member has been arranged at the distal end and a connecting member at the proximal end. Via the connecting member for instance a gas or liquid under pressure can be supplied to the balloon in order to expand the latter.

WO-A-94/24962 discloses a balloon catheter according to the preamble of claim 1.

The invention consists of a catheter as defined in claim 1.

When treating a patient, light can be supplied with this balloon catheter according to the invention, from the light-absorbing section at the proximal end to the location of the distal end of the catheter. There is in particular a need for this when fitting a stent made of a plastic material, curing due to the action of light, inside the patient. The folded stent is manoeuvred, together with the catheter, to the required position. Next the stent will be expanded and, in expanded state, cured by means of irradiation. When cured, the catheter is removed and the stent will remain, in expanded state, behind inside the vessel, for instance the blood vessel inside of which it has been fitted, in order to keep it patent.

The braided layer of light-conductive fibres constitutes at the same time a reinforcement with which desired properties of the basic body, such as for example the torsion stiffness, can be influenced. Different fibres, such as for instance thin metal wires, can of course also be incorporated in the braided layer in order to promote the required properties.

If the basic body has been made in such a way that a coating has been applied to the braided layer, this coating is preferably stripped of a section of the tubular member situated inside the balloon member, so that this forms the light-emitting end-section.

In order to guarantee uniform irradiation of the balloon and consequently of the stent arranged around it, the measure as set out in claim 2 is preferably employed.

The light-conductive material used when manufacturing the catheter according to the invention is preferably UV-light- conductive. UV-curing materials are suitable types of material for making plastic stents which are to cure due to the action of light.

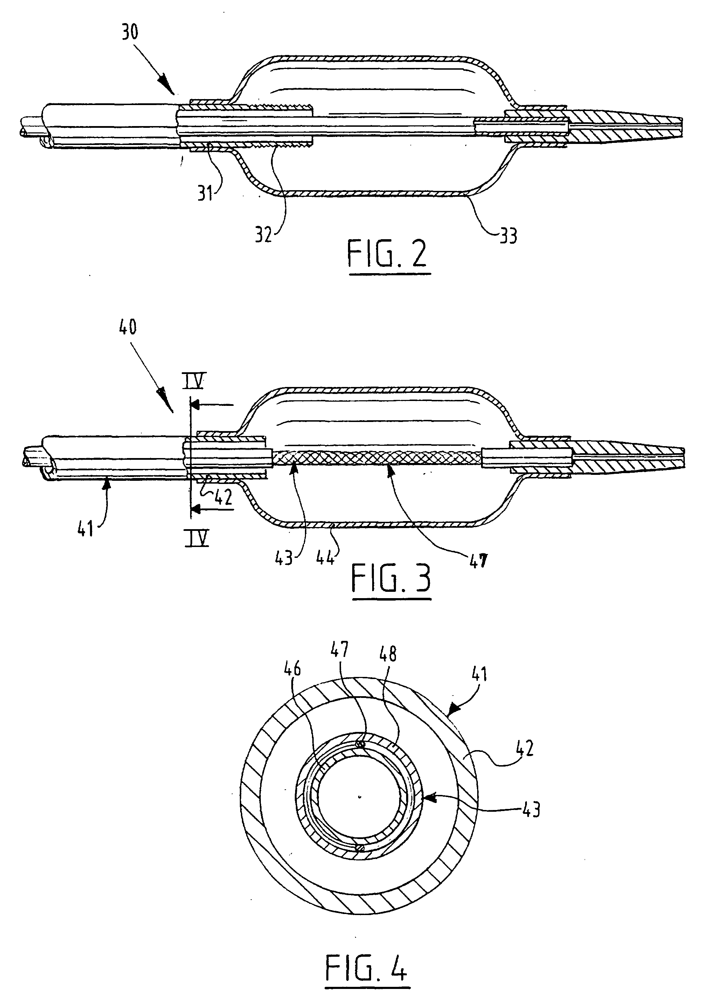

The invention will be explained in greater detail in the following description with reference to the attached figures 3 and 4.
- Figure 1: shows a partly cut away view of a catheter which is not part of the invention.
- Figure 2: illustrates the end-section of a catheter which is not part of the invention.
- Figure 3: represents a view of an embodiment of the catheter of the invention, similar in some aspects to that of figure 2.
- Figure 4: is a cross-section along the line IV-IV of figure 3.

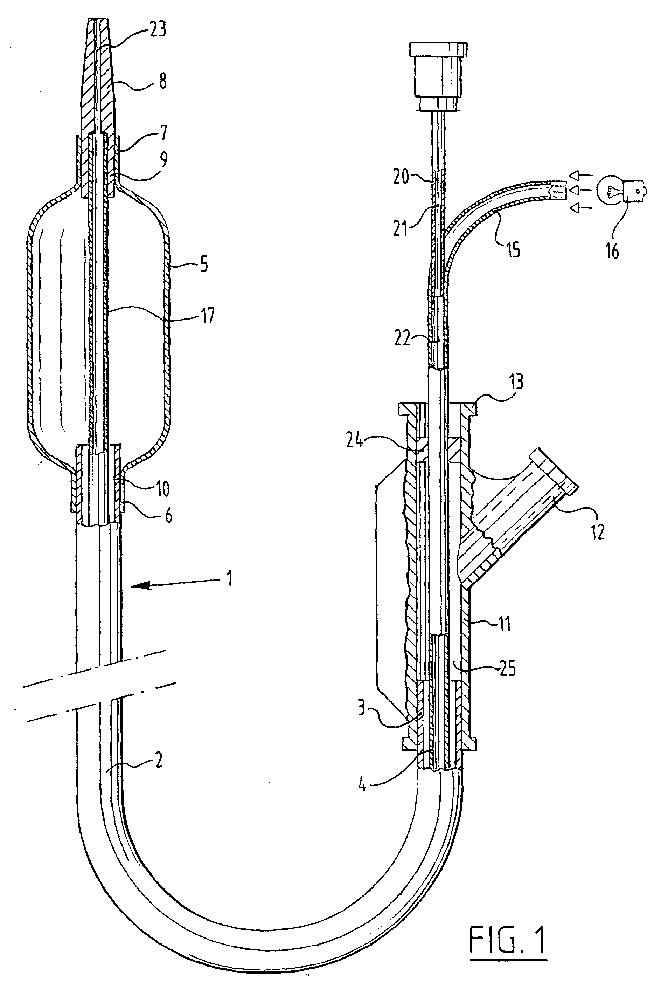

The catheter 1 shown in figure 1 comprises a basic body 2 which is made up of an outer tube-like member 3 and an inner tube-like member 4 extending through a lumen thereof. A balloon member 5 has been arranged on the distal end of the basic body 2. This balloon member 5 has been fixed with a relatively proximal end-section 6 to the outer tube-like member 3.

The inner tube-like member 4 extends further in a distal direction than the outer tube-like member 3, as far as a catheter-end-section 8 inside of which it is connected with the end-section 9.

The relatively distal end-section 7 of the balloon member 5 is fixed on the catheter-end-section 8.

At the proximal end of the basic body 2 a connecting member 11 has been arranged. This connecting member 11 comprises a connection 12 which is connected with a channel 25 which is formed by the remaining cross-section between the inner tube-like member 4 and the wall of the lumen of the outer tube-like member 3. Via this connection liquid or gas under pressure can be supplied to or removed from the balloon member 5 in order to make the latter expand or contract respectively.

The inner tube-like member 4 has been guided out of the connecting member 11 via a connection 13. At the site of the connection 13 the channel 25 has been closed off by means of a seal 24.

With the catheter illustrated, the inner tube-like member 4 has been made of a light-conductive plastic material in the form of a tube-like extrusion profile. The end-section of the tube-like member 4 which has been guided out of the connecting member 11, forms a light-absorbing end-section 15, into which light can be admitted by means of a, schematically indicated, source of light 16. The light passed into the light-absorbing section 15 by the source of light 16 is conducted through this tube-like member 4 to the distal end where the tube-like member 4 forms a light-emitting section 17. In order to conduct the light admitted to the light-emitting section 17, the outer wall of the inner tube-like member has been given a smooth finish. At the light-emitting end-section 17 situated inside the balloon member 5, this tube-like member has been roughened, for instance by grinding. As a result the light will be emitted in a radial direction and irradiate the inside of the balloon member 5. As the balloon member 5 has, in the usual manner, been made of a translucent material, the direct surroundings of the balloon member 5 will be irradiated if, by means of a source of light 16, light is introduced. Thus a stent, made of a material curing due to the action of light, which has been arranged around the balloon member 5, can be cured.

At the proximal end of the catheter 1 a connecting element 20 has been received inside the lumen 22 of the inner tube-like member 4. This connecting element 20 has a lumen 21 which is thus connected with the lumen 22 of the inner tube-like member 3. Via the connection 20 a guide wire can be introduced into the lumen 22. At the distal end the lumen 22 connects to a channel 23 inside the catheter-end-section 8, so that the guide wire can extend through the entire length of the catheter and the catheter can be advanced by passing it over this guide wire.

At the proximal end, the inner tube-like member can also be manufactured in such a way that the light will be admitted all round the circumference, so that a separate connection, such as the connection 20, is superfluous.

The catheter 30, partly illustrated in figure 2, comprises a basic body with an outer tube-like member 31 which has been made so as to be light-conductive. The end-section 32 of the outer tube-like member, situated inside the balloon member 33, has been manufactured in such a way that the light admitted at the proximal end is emitted. Thus, the inside of the balloon member 33, and in particular the surrounding area, is irradiated in a similar manner.

The outer tube-like member 31 can obviously extend as far as the relatively distal end of the balloon member 33. In the wall of the outer tube-like member 31, one or more holes are arranged for supplying and removing medium under pressure, for the purpose of expanding the balloon member.

With the embodiment of the catheter 40 as illustrated in figure 3 the basic body 41 again comprises an outer tube-like member 42 and an inner tube-like member 43. A balloon member 44 has been arranged in the manner described above.

The inner tube-like member 43 comprises a braided layer 47. As can be seen in particular in the cross-section of figure 4, the inner tube-like member 43 has been made up of an inner layer of plastic material 46 surrounded by a braided reinforcing layer 47, which in its turn is surrounded by a coating of a plastic material 48.

The layer 47 has been braided of light-conductive fibres which can conduct the light from the proximal end of the catheter to the distal end.

The section of the tube-like member 43 situated inside the balloon member 44 has been stripped of its outer coating 48, so that at that point the braided layer 47 is uncovered. The light admitted at the proximal end can thus be emitted inside the balloon member 44.

Obviously also the outer tube-like member can be manufactured in a similar manner so as to comprise a braided layer of light-conductive fibres, in which case this outer tube-like member extends into the balloon member where, if applied, it is stripped of its coating.

Because the light-conductive material has been received in the basic construction of the basic body, the catheter according to the invention can be manufactured so as to have the same dimensions as a similar catheter without light-conductive properties. In particular the cross-section of the basic body can thus remain limited.

## Claims

1. Catheter (40) comprising a tubular body (41) with a distal and a proximal end and a balloon member (44) arranged at the distal end, a light-receiving section at the proximal end, a light-emitting section at the distal end, both sections connected to each other by the body (41), the body (41) being at least partially light-conductive, and comprising an outer tubular member (42) and an inner tubular member (43), **characterized in that** at least one of said tubular members comprises a braided layer (47) with light-conductive fibres.

2. Catheter as claimed in claim 1, wherein a coating (48) is placed on top of the braided layer (47) with exception of a section of the situated inside the balloon member (44), thereby forming the light emitting section (17).

3. Catheter as claimed in claim 1 or 2, wherein the light-emitting section emits light substantially in a radial direction.

4. Catheter as claimed in one of the previous claims, wherein the boby (41) is UV-light-conductive.

## Patentansprüche

1. Katheter (40) umfassend einen röhrenförmigen Körper (41) mit einem distalen und einem proximalen Ende und ein am distalen Ende angeordnetes ballonförmiges Teil (44), einen Licht-aufnehmenden Abschnitt am proximalen Ende und einen Licht-emitierenden Abschnitt am distalen Ende, wobei beide Abschnitte durch den Körper (41) miteinander verbunden sind, wobei der Körper (41) zumindest teilweise lichtleitend ist und ein äußeres röhrenförmiges Teil (42) und ein inneres röhrenförmiges Teil (43) umfaßt, **dadurch gekennzeichnet, daß** zumindest einer der röhrenförmigen Teile eine gewickelte Schicht (47) mit lichleitenden Fasern umfaßt.

2. Katheter nach Anspruch 1, bei welchem eine Schicht (48) auf die gewickelte Schicht (47) plaziert wird, mit Ausnahme eines Abschnitts im Inneren des ballonförmigen Teils (44), wodurch der Licht-emittierende Abschnitt gebildet wird.

3. Katheter nach einem der Ansprüche 1 oder 2, bei welchem der Licht-emittierende Abschnitt Licht im Wesentlichen in radiale Richtung emittiert.

4. Katheter nach einem der voranstehenden Ansprüche, bei welchem der Grundkörper (41) UV-Licht-leitend ist.

## Revendications

1. Cathéter (40) comprenant un corps tubulaire (41) avec une extrémité distale et proximale et un élément formant ballonnet (44) placé au niveau de l'extrémité distale, une partie recevant la lumière au niveau de l'extrémité proximale, une partie émettant une lumière au niveau de l'extrémité distale, les deux parties étant reliées par le corps (41), le corps (41) conduisant au moins partiellement la lumière, et comprenant un élément tubulaire extérieur (42) et un élément tubulaire intérieur (43), **caractérisé en ce qu'**au moins un desdits éléments tubulaires comprend une couche tressée (47) composée de fibres conduisant la lumière.

2. Cathéter selon la revendication 1, dans lequel un revêtement (48) est placé sur la couche tressée (47), sauf sur une partie de celle-ci située à l'intérieur de l'élément formant ballonnet (44), formant ainsi la partie émettant la lumière (17).

3. Cathéter selon la revendication 1 ou 2, dans lequel la partie émettant la lumière émet de la lumière essentiellement dans une direction radiale.

4. Cathéter selon l'une des revendications précédentes, dans lequel le corps (41) conduit la lumière UV.
